# FASCICULE DE BREVET EUROPEEN

(11) **EP 1 107 766 B1**
(45) Date de publication et mention de la délivrance du brevet: **19.03.2003**
(21) Numéro de dépôt: 99941691.0
(22) Date de dépôt: 03.09.1999
(51) Int. Cl.: A61K 33/00, A61P 31/12

(54) **UTILISATION DU GLUCONATE DE LITHIUM DANS LA FABRICATION D'UN MEDICAMENT POUR LE TRAITEMENT DE L'HERPES ET DE LA DERMITE SEBORRHEIQUE**
VERWENDUNG VON LITHIUM-GLUCONAT ZUR HERSTELLUNG EINES ARZNEIMITTELS ZUR BEHANDLUNG VON HERPES UND SEBORRHOISCHER DERMATITIS
USE OF LITHIUM GLUCONATE IN THE PREPARATION OF A MEDICAMENT FOR TREATING HERPES AND SEBORRHEIC DERMATITIS

(30) Priorité: 03.09.1998 FR 9811025
(43) Date de publication de la demande: 20.06.2001
(73) Titulaire: LABCATAL, 92120 Montrouge (FR)
(72) Inventeur: BODIN, Jacques, F-92300 Levallois-Perret (FR); DRENO, Brigitte, F-44000 Nantes (FR)
(74) Mandataire: L'Helgoualch, Jean
(86) Numéro de dépôt international: FR9902108
(87) Numéro de publication internationale: WO00013649

(56) Documents cités:
- EP-A- 0 132 126
- EP-A- 0 132 384
- EP-A- 0 484 112
- WO-A-94/20119
- LANGTRY JA ET AL: "Topical lithium succinate ointment (Efalith) in the treatment of AIDS-related seborrhoeic dermatitis." CLIN EXP DERMATOL, SEP 1997, 22 (5) P216-9, XP002105249 ENGLAND
- AMSTERDAM JD ET AL: "Suppression of herpes simplex virus infections with oral lithium carbonate--a possible antiviral activity." PHARMACOTHERAPY, NOV-DEC 1996, 16 (6) P1070-5, XP002105250 UNITED STATES
- CUELENAERE C ET AL: "Use of topical lithium succinate in the treatment of seborrhoeic dermatitis [see comments]" DERMATOLOGY, 1992, 184 (3) P194-7, XP002105251 SWITZERLAND
- "A double-blind, placebo-controlled, multicenter trial of lithium succinate ointment in the treatment of seborrheic dermatitis. Efalith Multicenter Trial Group." J AM ACAD DERMATOL, MAR 1992, 26 (3 PT 2) P452-7, XP002105252 UNITED STATES
- LEEMING JP ET AL: "Lithium succinate and seborrhoeic dermatitis: an antifungal mode of action? [letter]" BR J DERMATOL, MAY 1990, 122 (5) P718-9, XP002105253 ENGLAND
- BOYLE J ET AL: "Use of topical lithium succinate for seborrhoeic dermatitis." BR MED J (CLIN RES ED), JAN 4 1986, 292 (6512) P28, XP002105254 ENGLAND
- RYBAKOWSKI J. ET AL: "Lithium succinate ointment in topical treatment of herpes simplex infections" LITHIUM_(_LITHIUM_), 2/2 (117-118), XP002105255 United Kingdom
- LEEMING J.P. ET AL: "Use of topical lithium succinate in the treatment of seborrhoeic dermatitis (1)" DERMATOLOGY_(_DERMATOLOGY_), 187/2 (149-150), XP002105256 Switzerland
- ROTH R.: "Topical treatment of seborrheic dermatitis with zinc sulfate and lithium succinate" TW DERMATOLOGIE_(_TW DERMATOL._), 24/2 (130-133), XP002105257 Germany
- "Seborrheic dermatitis: Lithium deprives the growth basis of yeast fungus" THERAPIEWOCHE_(_THERAPIEWOCHE_), 44/18 (1032-1033), XP002105258 Germany
- FRITZ K. ET AL: "Topical application of 8% lithium succinate und 0.05% zincsulfate in seborrhoic Dermatitis" H+G ZEITSCHRIFT FUR HAUTKRANKHEITEN_(_H G Z. HAUTKR._), 70/2 (155-156), XP002105259 Germany
- ELSNER P.: "Topical lithium succinate in the treatment of seborrheic dermatitis: Results of the Swiss multicenter phase IV study" AKTUELLE DERMATOLOGIE_(_AKTUEL. DERMATOL._), 22/12 (340-344), XP002105260 Germany
- HORROBIN, D. F.: "Lithium, fatty acids and seborrheic dermatitis: a new mechanism of lithium action and a new treatment for seborrheic dermatitis" LITHIUM, 1990, 1, 149-55, XP002105261
- WRIGHT S ET AL: "TOPICAL LITHIUM IN THE TREATMENT OF ITCH ASSOCIATED WITH SEBORRHEIC DERMATITIS NODULAR PRURIGO AND UREMIA" INTERNATIONAL SYMPOSIUM ON ITCH: BASIC AND CLINICAL ASPECTS, STOCKHOLM, SWEDEN, MAY 17-19, 1990. SKIN PHARMACOL, vol. 2, no. 4, 1990, pages 233-4, XP002105262
- HORROBIN D F: "LITHIUM EFFECTS ON FATTY ACID METABOLISM AND THEIR ROLE IN THERAPY OF SEBORRHEIC DERMATITIS AND HERPES INFECTIONS" SCHRAUZER, G. N. AND K.-F. KLIPPEL (ED.). LITHIUM IN BIOLOGY AND MEDICINE: NEW APPLICATIONS AND DEVELOPMENTS;INTERNATIONAL SYMPOSIUM ON PRESENT STATUS AND PERSPECTIVES OF LITHIUM IN BIOLOGY AND MEDICINE, TRIER, GERMANY, MAY 25-27, 1990. XIII+209P. VC, XP002105263
- WINTHER M D ET AL: "EFFICACY OF TOPICAL PREPARATIONS CONTAINING LITHIUM AGAINST HERPES SIMPLEX VIRUS INFECTIONS IN THE MOUSE" THE FIFTH INTERNATIONAL CONFERENCE ON ANTIVIRAL RESEARCH, VANCOUVER, BRITISH COLUMBIA, CANADA, MARCH 8-13, 1992. ANTIVIRAL RES, vol. 17, no. suppl.1, 1992, page 115 XP002105264
- HORROBIN D F: "LITHIUM AND ESSENTIAL FATTY ACID METABOLISM IN PSYCHIATRIC DISORDERS HERPES VIRUS INFECTIONS AND SEBORRHOEIC DERMATITIS" BIRCH, N. J. (ED.). LITHIUM AND THE CELL: PHARMACOLOGY AND BIOCHEMISTRY. XIV+351P. ACADEMIC PRESS LTD.: LONDON, ENGLAND, UK;SAN DIEGO, CALIFORNIA, USA. ILLUS. ISBN 0-12-099300-7., XP002105265
- SKINNER GR: "Lithium ointment for genital herpes [letter]" LANCET, JUL 30 1983, 2 (8344) P288, XP002105266 ENGLAND

## Description

La présente invention concerne l'application du gluconate de lithium en thérapeutique, et plus particulièrement l'utilisation du gluconate de lithium dans la préparation d'un médicament administré par voie topique et destiné au traitement de l'herpès et de la dermite séborrhéique, ainsi qu'une composition adaptée à un tel traitement.

On sait que certains sels de lithium peuvent être utilisés en thérapeutique pour le traitement des troubles psychiques. Plus particulièrement, on connaît des spécialités contenant du gluconate de lithium, utilisées par voie orale pour le traitement de la psychose maniaco dépressive (voir dictionnaire Vidal, Editions OVP, Paris 1998). Ces spécialités (par exemple Neurolithium® 1g/5ml et Neurolithium® 2g/10ml) permettent d'obtenir des lithémies de l'ordre de 0,5 à 0,8 m.mol/l, mais des effets indésirables sont parfois constatés à ces concentrations.

D'autre part, certains sels de lithium ont été décrits comme possédant une activité utile dans le traitement de certaines affections dermatologiques. Ainsi, le brevet EP-A-484.112 indique que le lithium, sous forme de succinate, de chlorure ou de carbonate, peut être utilisé par voie topique pour le traitement de patients atteints de Molluscum contagiosum, à des concentrations en ions lithium comprises entre 0,3 et 2 %.

La dermite séborrhéique est une dermatose érythématosquameuse rencontrée fréquemment chez les adultes jeunes. Elle se manifeste par un érythème et une desquamation à des degrés divers selon la gravité de l'affection, apparaissant par périodes de poussées suivies de rémissions plus ou moins longues, généralement sur le visage, le cuir chevelu ou le thorax. Les traitements connus se font au moyen de compositions administrées par voie topique, contenant des antifongiques tels que des dérivés de l'imidazole, ou des antiinflammatoires tels que des dermocorticoïdes. Cependant, l'efficacité de ces traitements est très incertaine.

L'infection par le virus de l'herpès simplex non génital, primaire ou récidivante, se manifeste cliniquement par un bouquet de vésicules surmontant une plaque érythémateuse, sur la peau et sur les muqueuses. L'herpès génital est une maladie virale sexuellement transmissible qui se caractérise par l'apparition de vésicules en bouquet au site d'inoculation, associée à une adénopathie régionale lors de la primo-infection. La caractéristique de l'affection est la récidive de vésicules au même endroit.

Les traitements locaux usuels de l'herpès se font généralement au moyen de compositions pharmaceutiques administrées par voie topique, contenant de l'acyclovir, de la trifluorothymidine ou de la vidarabine phosphate disodique.

Les travaux de recherche effectués par la demanderesse ont permis de démontrer que le gluconate de lithium pouvait être utilisé efficacement dans le traitement de l'herpès et de la dermite séborrhéique en évitant les effets secondaires mentionnés ci-dessus.

Ainsi la présente invention a pour objet l'utilisation du gluconate de lithium dans la préparation d'un médicament administré par voie topique et destiné au traitement de l'herpès et de la dermite séborrhéique.

L'invention a également pour objet une nouvelle composition à base de gluconate de lithium utilisable en thérapeutique, présentée sous une forme administrable par voie topique, destinée au traitement de l'herpès et de la dermite séborrhéique, et spécialement formulée pour ce traitement.

Selon la présente invention, la teneur en gluconate de lithium dans la composition est comprise entre 1 % et 50 % en poids, et de préférence entre 5 % et 20 %, par rapport au poids total de la composition. L'utilisation d'une forme topique permet de s'affranchir des problèmes d'effets secondaires mentionnés ci-dessus, en délivrant in situ la quantité de produit nécessaire à son activité, sans passage systémique significatif.

Comme indiqué ci-dessus, suivant l'invention la concentration en gluconate de lithium peut être comprise entre 1 et 50 %, ce qui permet de libérer dans l'épiderme des quantités de lithium de l'ordre de 5 à 100 mM sans passage systémique significatif.

La composition suivant l'invention contient de préférence du gluconate de lithium sous forme ionisée, en association avec un ou plusieurs excipients aqueux.

Les études effectuées en utilisant des compositions suivant la présente invention ont mis en évidence une meilleure efficacité et une meilleure tolérance par rapport aux compositions connues, en particulier les compositions à base d'autres sels de lithium tels que le succinate, le chlorure ou le carbonate.

L'action virucide du gluconate de lithium sur les virus de l'herpès HSV-1 et HSV-2 (herpes simplex virus) a été vérifiée. On a ainsi constaté qu'une solution à 25 mM/litre de gluconate de lithium permet de faire diminuer le titre d'une suspension de virus herpétique d'un facteur de 10⁷ pour HSV-1 et de 10⁹ pour HSV-2.

Le gluconate de lithium agirait à des concentrations de l'ordre de 50 mM en inhibant la formation de l'acide arachidonique et de l'acide dihommogammalinoléique précurseurs des médiateurs de l'inflammation (prostaglandine E1, prostaglandine E2, thromboxane A2).

On a également observé que le lithium, et plus particulièrement le lithium ionisé, inhibe la croissance de Mallassezia furfur (levure responsable ou conséquence de l'apparition de la dermite séborrhéique), directement à des concentrations de l'ordre de 1,5 à 100 mM.

De plus, il semble agir indirectement par inhibition de la libération des acides gras nécessaires à la croissance de cette levure.

Il peut paraître surprenant que la même composition conforme à l'invention soit efficace à la fois pour le traitement de l'herpès et pour le traitement de la dermite séborrhéique alors que les pathologies de ces deux affections sont sensiblement différentes. Ceci constitue un avantage supplémentaire de la présente invention.

La composition suivant l'invention peut être présentée sous forme de gel, de lotion, de collyre, de crème ou de toute autre forme usuelle en application topique, la teneur en gluconate étant comprise entre 1 % et 50 % en poids, et de préférence entre 5 % et 20 % en poids. Le pH de la composition peut varier selon la forme de la composition, et il est généralement compris entre 6 et 8, et de préférence voisin de 6,5 à 7,5.

La composition peut contenir, outre le gluconate de lithium, de préférence le gluconate de lithium ionisé, des excipients adaptés en fonction de l'usage envisagé, tels que des agents hydratants, par exemple le propylène glycol ou le glycérol, des agents gélifiants, par exemple un carbomère ou un dérivé de cellulose, un agent neutralisant destiné à ajuster le pH à la valeur voulue, par exemple l'acide citrique, la triéthanolamine ou l'hydroxyde de sodium, un conservateur, par exemple le p-hydroxybenzoate de méthyle ou de propyle, le phénoxyéthanol ou le chlorure de benzalkonium, un tensioactif moussant, par exemple le laurylsulfate de sodium, un stabilisant, par exemple le diéthanolamide d'acide gras de coprah, un solvant, par exemple l'eau purifiée, le polyéthylène glycol ou le myristate d'isopropyle. D'autres excipients et additifs usuels peuvent être utilisés en fonction du mode d'administration envisagé.

L'efficacité du gluconate de lithium en gel à 8 % dans le traitement de la dermite séborrhéique a été vérifiée par une étude clinique randomisée contre placebo en double aveugle sur 129 patients. Les résultats obtenus démontrent une efficacité significative sur l'érythème et la desquamation, avec une rémission complète au bout de quatre à huit semaines dans la majorité des cas. De plus, cette étude montre que le gluconate de lithium, administré conformément à la présente invention, s'avère plus efficace qu'une composition connue à base de succinate de lithium alors pourtant que la teneur en élément lithium est trois fois moindre (une composition à 8 % de gluconate de lithium correspond à 0,274 % en élément lithium tandis qu'une composition à 8 % de succinate de lithium correspond à 0,855 % en élément lithium).

Dans le traitement de l'herpès, l'efficacité du gluconate de lithium en gel à 8 % a été vérifiée par une étude ouverte sur 30 patients atteints d'herpès labial.

La posologie est déterminée par le médecin en fonction de l'affection, de son degré de gravité, et de la condition du patient. Dans le cas de la dermite séborrhéique, elle est généralement de 1 ou 2 applications par jour pendant 3 à 8 semaines. Dans le cas de l'herpès, la composition est appliquée de préférence 3 à 4 fois par jour dès l'apparition de la poussée herpétique, puis consécutivement pendant 5 à 10 jours.

Des exemples de réalisation sont donnés ci-après pour illustrer l'invention.

### Exemple 1

### Formulation 1 : Gel

Un gel incolore ayant la composition suivante est préparé par les techniques usuelles :

| | | |
|---|---|---|
| Gluconate de lithium | | 8,0 g |
| Glycérine | | 10,0 g |
| Carbomère | | 1,5 g |
| Hydroxyde de sodium | q.s.p. | pH 6,8 |
| Parahydroxybenzoate de méthyle | | 0,10 g |
| Parahydroxybenzoate de propyle | | 0,05 g |
| Propylène glycol | | 5,0 g |
| Eau purifiée | q.s.p. | 100,0 g |

Cet exemple de formulation correspond à une teneur de 0,27 % en ion lithium, et constitue une forme préférentielle de composition selon l'invention.

Cette composition est plus particulièrement destinée au traitement de la dermite séborrhéique. Les tests effectués ont montré que, appliquée deux fois par jour pendant quatre semaines sur la zone de la peau à traiter, elle entraîne une disparition presque complète de la dermite, sans entraîner d'effet secondaire.

Une étude in vitro effectuée sur des échantillons de peau abdominale humaine a montré que l'absorption percutanée du gluconate de lithium, utilisé sous la forme de la composition ci-dessus, peut être considérée comme négligeable. En effet, après application d'une quantité moyenne de 109 mg de gel de composition ci-dessus sur des échantillons de peau, dans des conditions proches de celles de l'utilisation thérapeutique, après un contact de 48 heures, les quantités retrouvées dans l'épiderme et dans le derme sont respectivement de 0,20 % et 0,13 % de la quantité appliquée sur la peau.

Cette étude montre que la quantité de gluconate de lithium susceptible de traverser la barrière cutanée est négligeable et que les risques éventuels liés à la toxicité du lithium sont insignifiants.

Ceci est confirmé par la mesure des lithémies effectuées pendant l'étude clinique mentionnée plus haut.

### Exemple 2

### Formulation 2 : Lotion

On prépare une lotion pour application dermique ayant la composition indiquée ci-après :

| | | |
|---|---|---|
| Gluconate de lithium | | 5,0 g |
| Acide citrique | q.s.p. | pH 6,8 |
| Parahydroxybenzoate de méthyle | | 0,10 g |
| Parahydroxybenzoate de propyle | | 0,05 g |
| Eau purifiée | q.s.p. | 100,0 ml |

Cette lotion est utilisée en applications deux fois par jour pendant quatre à cinq semaines, par des patients affectés de dermite séborrhéique. Au terme de ce traitement, on constate une disparition presque complète de la dermite séborrhéique.

### Exemple 3

### Formulation 3 - Collyre

Un collyre ayant la composition indiquée ci-après, est préparé par les techniques usuelles :

| | | |
|---|---|---|
| Gluconate de lithium | | 3,25 g |
| Hydroxypropylméthyl cellulose | | 0,2 g |
| Acide borique | q.s.p. | pH 7,4 |
| Chlorure de benzalkonium | | 0,01 g |
| Eau purifiée | q.s.p. | 100,0 ml |

Ce collyre est appliqué trois à quatre fois par jour pendant quelques jours, chez des patients souffrant d'herpès oculaire, jusqu'à cicatrisation complète de l'affection.

### Exemple 4

### Formulation 4 : Crème

En utilisant les techniques usuelles, on prépare une crème ayant la composition suivante :

| | | |
|---|---|---|
| Gluconate de lithium | | 9,0 g |
| Stéarate de diéthylène glycol | | 6,0 g |
| Huile de vaseline | | 6,0 g |
| Glycérine | | 6,0 g |
| Acide stéarique | | 6,0 g |
| Acide citrique | q.s.p. | pH 7,0 |
| Parahydroxybenzoate de méthyle | | 0,10 g |
| Parahydroxybenzoate de propyle | | 0,05 g |
| Eau purifiée | q.s.p. | 100,0 ml |

Cette crème est appliquée trois à quatre fois par jour sur la zone de la peau affectée d'un patient souffrant d'une poussée d'herpès labial, pendant cinq à huit jours, c'est-à-dire pendant les phases de primo-infection et les récurrences ultérieures. On constate alors une réduction significative de l'infection par comparaison avec des patients recevant un placebo.

### Exemple 5

### Formulation 5 : Crème

| | | |
|---|---|---|
| Gluconate de lithium | | 4,0 g |
| Glycérine | | 6,0 g |
| Paraffine solide | | 2,0 g |
| Huile de vaseline | | 6,85 g |
| Perhydrosqualène | | 1,5 g |
| Cétéaryl glucoside | | 5,0 g |
| Parahydroxybenzoate de méthyle | | 0,10 g |
| Parahydroxybenzoate de propyle | | 0,05 g |
| Eau purifiée | q.s.p. | 100,0 ml |

Cette crème est utilisée en applications quotidiennes, comme celle de l'exemple 4 ci-dessus.

### Exemple 6

### Formulation 6 : Crème

| | | |
|---|---|---|
| Gluconate de lithium | | 15,0 g |
| Cire autoémulsionnable | | 15,0 g |
| Triglycéride d'acide gras à chaîne moyenne | | 10,0 g |
| 2-octyldodécanol | | 4,0 g |
| Acide citrique | q.s.p. | pH 6,8 |
| Parahydroxybenzoate de méthyle | | 0,10 g |
| Parahydroxybenzoate de propyle | | 0,05 g |
| Eau purifiée | q.s.p. | 100,0 ml |

Cette crème est utilisée en applications quotidiennes, comme celle de l'exemple 4 ci-dessus.

### Exemple 7

### Formulation 7 : Gel moussant

| | | |
|---|---|---|
| Gluconate de lithium | | 8,0 g |
| Lauryléthersulfate de sodium | | 25,0 g |
| Diéthanolamide d'acide gras de coprah | | 2,5 g |
| Acide citrique | q.s.p. | pH 6,8 |
| Parahydroxybenzoate de méthyle | | 0,10 g |
| Parahydroxybenzoate de propyle | | 0,05 g |
| Eau purifiée | q.s.p. | 100,0 ml |

Ce gel est utilisé en applications sur le cuir chevelu deux à trois fois par semaine, pendant quatre à cinq semaines, par des patients atteints de dermite séborrhéique. Au terme du traitement, on constate que les signes de dermite ont totalement disparu.

D'autres compositions à base de gluconate de lithium associé à des excipients usuels peuvent être préparées par les techniques connues.

## Revendications

1. Utilisation du gluconate de lithium dans la préparation d'un médicament administré par voie topique et destiné au traitement de l'herpès et de la dermite séborrhéique.

2. Utilisation selon la revendication 1, **caractérisée en ce que** le gluconate de lithium est sous forme ionisée.

3. Utilisation selon l'une quelconque des revendications 1 et 2, **caractérisée en ce que** le gluconate de lithium est associé à un excipient aqueux.

4. Utilisation selon l'une quelconque des revendications précédentes, **caractérisée en ce que** la composition est administrée sous forme de gel, de lotion, de crème ou de collyre.

5. Composition à base de gluconate de lithium utilisable en thérapeutique, présentée sous une forme administrable par voie topique, destinée au traitement de l'herpès et de la dermite séborrhéique, **caractérisée en ce que** la teneur en gluconate de lithium est comprise entre 1 % et 50 % en poids par rapport au poids total de la composition.

6. Composition selon la revendication 5, **caractérisée en ce que** la teneur en gluconate de lithium est comprise entre 5 % et 20 % en poids.

7. Composition selon l'une quelconque des revendications 5 et 6, **caractérisée en ce qu'**elle contient du gluconate de lithium ionisé.

8. Composition selon l'une quelconque des revendications 5 à 7, **caractérisée en ce que** le gluconate de lithium est associé à un excipient aqueux.

9. Composition selon l'une quelconque des revendications 5 à 8, **caractérisée en ce qu'**elle est présentée sous forme de gel, lotion, collyre ou crème.

## Claims

1. Use of lithium gluconate in the preparation of a medicament administered by the topical route and intended for the treatment of herpes and of seborrheic dermatitis.

2. Use according to claim 1, **characterized in that** the lithium gluconate is in ionized form.

3. Use according to either of claims 1 and 2, **characterized in that** the lithium gluconate is combined with an aqueous excipient.

4. Use according to any one of the preceding claims, **characterized in that** the composition is administered in the form of a gel, lotion, cream or collyrium.

5. Composition based on lithium gluconate which can be used in therapy, presented in a form administrable by the copical route, intended for the treatment of herpes and of seborrheic dermatitis, **characterized in that** the content of lithium gluconate is between 1% and 50% by weight relative to the total weight of the composition.

6. Composition according to claim 5, **characterized in that** the content of lithium gluconate is between 5% and 20% by weight.

7. Composition according to either of claims 5 and 6, **characterized in that** it contains ionized lithium gluconate.

8. Composition according to any one of claims 5 to 7, **characterized in that** the lithium gluconate is combined with an aqueous excipient.

9. Composition according to any one of claims 5 to 8, **characterized in that** it is presented in the form of a gel, lotion, collyrium or cream.

## Patentansprüche

1. Verwendung von Lithiumgluconat bei der Herstellung eines topisch verabreichten Medikaments zur Behandlung von Herpes und seborrhoischer Dermatitis.

2. Verwendung nach Anspruch 1, **dadurch gekennzeichnet, dass** das Lithiumgluconat in ionisierter Form vorliegt.

3. Verwendung nach einem der Ansprüche 1 und 2, **dadurch gekennzeichnet, dass** das Lithiumgluconat mit einem wässrigen Exzipienten assoziiert ist.

4. Verwendung nach einem der vorangegangenen Ansprüche, **dadurch gekennzeichnet, dass** die Zusammensetzung in Gel-, Lotions-, Cremen- oder Augentropfen-Form verabreicht wird.

5. Therapeutisch einsetzbare Zusammensetzung auf Basis von Lithiumgluconat zur Behandlung von Herpes und seborrhoischer Dermatitis, die in auf topischem Weg verabreichbarer Form vorliegt, **dadurch gekennzeichnet, dass** der Gehalt an Lithiumgluconat zwischen 1 und 50 Gew.-%, bezogen auf das Gesamtgewicht der Zusammensetzung, beträgt.

6. Zusammensetzung nach Anspruch 5, **dadurch gekennzeichnet, dass** der Gehalt an Lithiumgluconat zwischen 5 und 20 Gew.-% beträgt.

7. Zusammensetzung nach einem der Ansprüche 5 und 6, **dadurch gekennzeichnet, dass** sie ionisiertes Lithiumgluconat enthält.

8. Zusammensetzung nach einem der Ansprüche 5 bis 7, **dadurch gekennzeichnet, dass** das Lithiumgluconat mit einem wässrigen Exzipienten assoziiert ist.

9. Zusammensetzung nach einem der Ansprüche 5 bis 8, **dadurch gekennzeichnet, dass** sie in Gel-, Lotions-, Augentropfen- oder Cremen-Form vorliegt.
